# EUROPEAN PATENT SPECIFICATION

(11) **EP 4 401 637 B1**
(45) Date of publication and mention of the grant of the patent: **25.11.2026**
(21) Application number: 22870593.5
(22) Date of filing: 14.09.2022
(51) Int. Cl.: A61B 5/291, A61B 5/369, A61B 34/10

(54) **SYSTEMS AND METHODS FOR STEREO-EEG IMPLANTATION AND RESECTION SURGERIES**
SYSTEME UND VERFAHREN ZUR STEREO-EEG-IMPLANTATION UND RESEKTIONSCHIRURGIE
SYSTÈMES ET MÉTHODES D'IMPLANTATION DE STÉRÉO-EEG ET DE CHIRURGIE DE RÉSECTION

(30) Priority: 15.09.2021 US 202163244327 P; 02.06.2022 US 202263348212 P
(43) Date of publication of application: 24.07.2024
(73) Proprietor: Duke University, Durham, North Carolina 27705 (US)
(72) Inventor: GRILL, Warren, Durham, North Carolina 27705 (US); THIO, Brandon, Durham, North Carolina 27705 (US)
(74) Representative: Harris, Oliver John Richard
(86) International application number: PCT/US2022/043430
(87) International publication number: WO 2023/043773

(56) References cited:
- EP-A1- 3 855 453
- WO-A1-2020/167997
- US-A1- 2003 093 004
- US-A1- 2017 087 367
- US-A1- 2018 055 394
- US-A1- 2018 310 854
- US-A1- 2021 169 417
- HYDE DAMON E ET AL: "Localization of stereo-electroencephalography signals using a finite difference complete electrode model", 2017 39TH ANNUAL INTERNATIONAL CONFERENCE OF THE IEEE ENGINEERING IN MEDICINE AND BIOLOGY SOCIETY (EMBC), IEEE, 11 July 2017 (2017-07-11), pages 3600 - 3603, XP033152811, DOI: 10.1109/EMBC.2017.8037636
- CAUNE V ET AL: "Evaluating dipolar source localization feasibility from intracerebral SEEG recordings", NEUROIMAGE, vol. 98, 2 May 2014 (2014-05-02), pages 118 - 133, XP028879187, ISSN: 1053-8119, DOI: 10.1016/J.NEUROIMAGE.2014.04.058
- SPARKS RACHEL ET AL: "Automated multiple trajectory planning algorithm for the placement of stereo-electroencephalography (SEEG) electrodes in epilepsy treatment", INTERNATIONAL JOURNAL OF COMPUTER ASSISTED RADIOLOGY AND SURGERY, SPRINGER, DE, vol. 12, no. 1, 1 July 2016 (2016-07-01), pages 123 - 136, XP036127546, ISSN: 1861-6410, [retrieved on 20160701], DOI: 10.1007/S11548-016-1452-X
- YUULI MIINALAINEN: "Numerical EEG Forward Modeling with Dipolar Sources: H(div) Approach", MASTER OF SCIENCE THESIS, 1 September 2017 (2017-09-01), pages 1 - 75, XP093062252

## Description

### GOVERNMENT FUNDING

This invention was made with Government support under Federal Grant No. UL1 TR002553 awarded by the National Institutes of Health Clinical and Translational Science Awards (CTSA). The Federal Government has certain rights to the invention.

### BACKGROUND

About 30% of the 65 million people with epilepsy worldwide do not benefit from pharmacological intervention and are candidates for surgical resection of the epileptogenic zone - a theoretical region of the brain where, if you can remove it, a patient will experience full seizure freedom. Proper resections rely on robust localizations of the epileptogenic zone using stereo-electroencephalogram ("stereo-EEG" or "sEEG")

Stereo-EEG is a minimally invasive technique where up to approximately 25 electrodes are implanted throughout the brain via small transcranial burr holes. Continuous recordings are obtained from the electrode contacts to allow clinicians to delineate the epileptogenic zone, the minimum amount of tissue needed to be resected to achieve seizure freedom. Currently, clinicians manually conduct each step of sEEG implantation planning and analysis, which is a subjective and time-consuming process. Hence, there is an ongoing opportunity for improvements to implantation planning and execution.

A paper by Hyde et al (2017 39th Annual International Conference of the IEEE. Engineering in Medicine and Biology Society (EMBC), IEEE. 3600-3603; XP033152811) discusses employing a model to localize activity from sEEG measurements in a patient undergoing resective surgery for the treatment of epilepsy.

### SUMMARY

The Summary is provided to introduce a selection of concepts that are further described below in the Detailed Description. This Summary is not intended to identify key or essential features of the claimed subject matter, nor is it intended to be used as an aid in limiting the scope of the claimed subject matter.

The present disclosure includes a method that reconstructs propagating neural activity with time-dependent stereo-EEG data received from a plurality of electrodes, the method comprising: simulating a static source reconstruction using the set of time-dependent stereo-EEG data; clustering adjacent populations of active neural sources together to generate time-dependent active clusters of neural sources; fitting a spatial trajectory to the time-dependent active clusters; constraining a search region to cortical points within a threshold distance of the spatial trajectory; simulating a propagating source reconstruction based on the search region and the spatial trajectory of the time-dependent active clusters; and displaying the propagating source in a three-dimensional head model.

In some embodiments, generating the three-dimensional head model comprises: receiving images of a patient brain; determining a coordinate for each of a plurality of electrodes; generating three-dimensional geometry of the patient brain based on the images; generating three-dimensional geometry of the plurality of electrodes within the three-dimension geometry of the patient brain based on the images and the coordinate for each of the plurality of electrodes; defining an electrical property of the three-dimensional geometry of the patient brain; and outputting a finite element model for the patient brain and the plurality of electrodes.

### SUMMARY OF THE INVENTION

The invention is as set out in the appended claims.

### BRIEF DESCRIPTION OF THE DRAWINGS

The accompanying figures are provided by way of illustration and not by way of limitation.

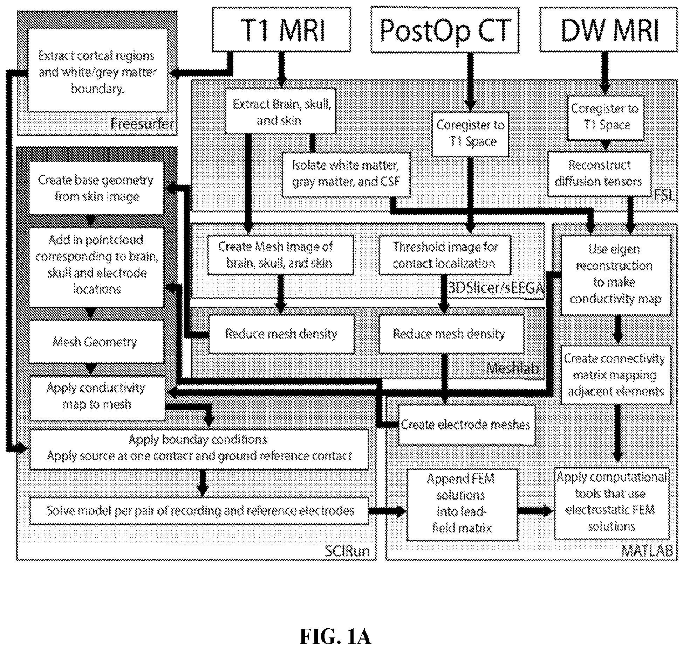

FIG. 1A is a diagram illustrating a method for creating patient-specific head models (e.g., a computational modeling pipeline). Neuroimaging (e.g., T1 MRI, PostOp CT, PreOp CT, and DW MRI) is input into the FMRIB Software Library ("FSL") to orient all the images in the same coordinate space. T1 MRI defines the base structure of the models, the CT images are used to localize the electrode contacts, and the DW MRI defines the anisotropic conductivity of the different brain tissues. All geometries and tissue properties are input into SCIRun (from the Center for Integrative Biomedical Computing) to solve for the electrode voltages generated by each potential brain source. Brain sources are defined using the Freesurfer (from the Laboratory for Computational Neuroimaging) defined white/grey matter boundary. The voltages are appended into a lead-field transfer matrix in MATLAB for post processing.
FIG. 1B is a diagram illustrating a method for creating patient-specific head models (e.g., a computational modeling pipeline).
FIG. 2 is an outline of a method determining and visualizing the recordable brain tissue for a patient by a set of sEEG electrodes. Models are developed using the computational modeling method of FIG. 1A. For a set of sEEG electrodes, the method iterates through each contact and determines the tissue that is recordable by a single electrode contact. The method then visualizes the recordable tissue of all the contacts. In the visualization, the color gradients represent the number of contacts that can record a certain area of cortex.
FIG. 3 is a 3D visualization of the recordable area for a set of 12 sEEG electrodes (contacts shown in pink). Color indicates how many electrodes can record from that area, according to the legend provided.
FIG. 4 is an outline of a method for sEEG electrode implantation trajectory optimization to maximize recordable brain tissue. Models are generated using the method of FIG. 1A, but electrodes are not defined in the model. Instead, the method solves for the voltages at every possible recording location in the head for every possible neural source. In some embodiments, a Monte Carlo tree search approach is used to determine the minimum number of necessary implanted electrodes to map a region of cortex.

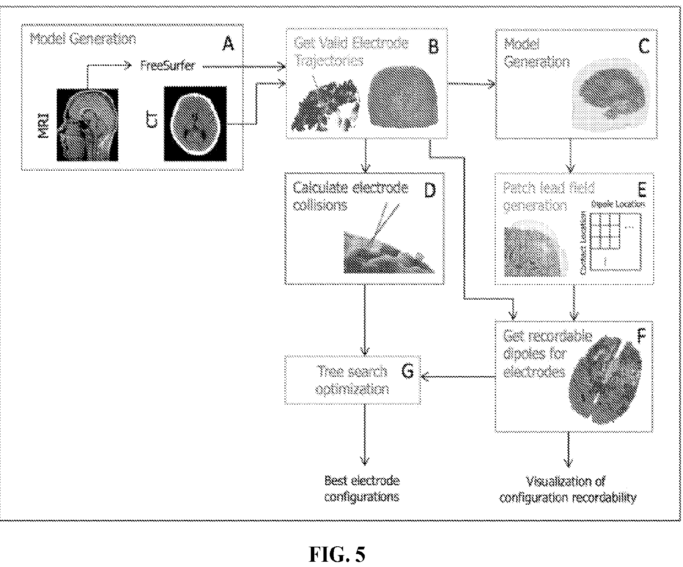

FIG. 5 is a diagram illustrating simulation-based sEEG electrode configuration optimization method. The illustrated method uses T1-weighted MRI, CT, and DW MRI imaging modalities at STEP A. At STEP B, valid electrode trajectories are generated by finding the valid insertion locations, varying insertion angle and depth, and excluding electrodes that intersect the sulci, for example. At STEP C, a finite element head model is generated with five tissue types, adding all valid electrode contact locations into the mesh. At STEP D, occurrences of collisions between all pair of valid electrodes are determined. At STEP E, the patch lead-field matrix is determined, which defines the transfer function between all electrode contact locations and an extended-dipole source model centered at every dipole in the cortical surface mesh. At STEP F, the patch lead field matrix is utilized to determine the recordable dipoles for all valid electrodes, which defines the recording sensitivity of an electrode position. At STEP G, the recording sensitivity is used to visualize the recordable area for any electrode configuration or in an optimized algorithm to find the configuration(s) that map the full region of interest (ROI) with the fewest number of electrodes.
FIG. 6 is graphs of maximum distance of discernible signal for an area of patch source.
FIG. 7 illustrates (top left) a table that shows the number of electrodes to map greater than 95% of a region of interest, which is smaller for smaller areas of cortex in the ROI and for larger dipole moment density estimates; (top right) an example of an electrode configuration to map the left temporal lobe; (bottom) graph of the percent ROI unmapped when a particular area of cortex needs to be mapped by one versus two electrodes.
FIG. 8 is a visualization comparing the method solution and the clinician-generated and implanted configuration, assuming a full cortex ROI.
FIG. 9 is a graph illustrating results from the method of FIG. 5, showing greater recording sensitivity at 100 µV thresholds for single mappings than the clinician-generated and implanted configuration, assuming a full cortex ROI.
FIG. 10 is a graph illustrating error in recordable dipoles using ADC compressed lead fields.
FIG. 11 is a diagram illustrating a method for optimization of sEEG electrode implantation trajectories to maximize recordable tissue.
FIG. 12 is an outline of a method for dynamic source reconstruction for sEEG.
FIG. 13 is an illustration of a reconstructed synthetic source.
FIG. 14 is a visualization of epileptiform zones for sEEG. Recordings of epileptiform activity is input to the dynamics source reconstruction method to determine the time courses and spatial extents of the neural sources. In some embodiments, each zone of reconstructed epileptiform activity is visualized on a mesh of the brain and allows a clinician to toggle the visibility of each region of interest.
FIG. 15 is an outline of a propagating source reconstruction method. Localizations are conducted using the sEEG recording includes the steps: static localization, patch clustering, trajectory fitting, space constraint, and reseeded localization.
FIG. 16 is graphs comparing reconstruction metrics for static and dynamic source reconstruction. (A) Jaccard loss. (B) Percent % overlap. (C) Localization error are shown for a single synthetic propagating source originating in the parietal lobe moving in the anterior to posterior direction in a single patient for 1000 time points. Larger values of Jaccard index and percent overlap are advantageous, and smaller values of localization error is advantageous.

### DETAILED DESCRIPTION

Section headings as used in this section and the entire disclosure herein are merely for organizational purposes and are not intended to be limiting.

### 1. Definitions

The terms "comprise(s)," "include(s)," "having," "has," "can," "contain(s)," and variants thereof, as used herein, are intended to be open-ended transitional phrases, terms, or words that do not preclude the possibility of additional acts or structures. The singular forms "a," "and" and "the" include plural references unless the context clearly dictates otherwise. The present disclosure also contemplates other embodiments "comprising," "consisting of" and "consisting essentially of," the embodiments or elements presented herein, whether explicitly set forth or not.

For the recitation of numeric ranges herein, each intervening number there between with the same degree of precision is explicitly contemplated. For example, for the range of 6-9, the numbers 7 and 8 are contemplated in addition to 6 and 9, and for the range 6.0-7.0, the number 6.0, 6.1, 6.2, 6.3, 6.4, 6.5, 6.6, 6.7, 6.8, 6.9, and 7.0 are explicitly contemplated. Recitation of ranges of values herein are merely intended to serve as a shorthand method of referring individually to each separate value falling within the range, unless otherwise-Indicated herein, and each separate value is incorporated into the specification as if it were individually recited herein. For example, if a concentration range is stated as 1% to 50%, it is intended that values such as 2% to 40%, 10% to 30%, or 1% to 3%, etc., are expressly enumerated in this specification. These are only examples of what is specifically intended, and all possible combinations of numerical values between and including the lowest value and the highest value enumerated are to be considered to be expressly stated in this disclosure.

"Subject" and "patient" as used herein interchangeably refers to any vertebrate, including, but not limited to, a mammal (e.g., cow, pig, camel, llama, horse, goat, rabbit, sheep, hamsters, guinea pig, cat, dog, rat, and mouse, a non-human primate (e.g., a monkey, such as a cynomolgus or rhesus monkey, chimpanzee, etc.) and a human). In some embodiments, the subject may be a human or a non-human. In one embodiment, the subject is a human. The subject or patient may be undergoing various forms of treatment.

"Treat," "treating" or "treatment" are each used interchangeably herein to describe reversing, alleviating, or inhibiting the progress of a disease and/or injury, or one or more symptoms of such disease, to which such term applies. Depending on the condition of the subject, the term also refers to preventing a disease, and includes preventing the onset of a disease, or preventing the symptoms associated with a disease. A treatment may be either performed in an acute or chronic way. The term also refers to reducing the severity of a disease or symptoms associated with such disease prior to affliction with the disease. Such prevention or reduction of the severity of a disease prior to affliction refers to administration of a treatment to a subject that is not at the time of administration afflicted with the disease. "Preventing" also refers to preventing the recurrence of a disease or of one or more symptoms associated with such disease.

"Therapy" and/or "therapy regimen" generally refer to the clinical intervention made in response to a disease, disorder or physiological condition manifested by a patient or to which a patient may be susceptible. The aim of treatment includes the alleviation or prevention of symptoms, slowing or stopping the progression or worsening of a disease, disorder, or condition and/or the remission of the disease, disorder or condition.

Unless otherwise defined herein, scientific and technical terms used in connection with the present disclosure shall have the meanings that are commonly understood by those of ordinary skill in the art. For example, any nomenclatures used in connection with, and techniques of, cell and tissue culture, molecular biology, neurobiology, microbiology, genetics, electrical stimulation, neural stimulation, neural modulation, and neural prosthesis described herein are those that are well known and commonly used in the art. The meaning and scope of the terms should be clear; in the event, however of any latent ambiguity, definitions provided herein take precedent over any dictionary or extrinsic definition. Further, unless otherwise required by context, singular terms shall include pluralities and plural terms shall include the singular.

### 2. Patient-Specific Head Modeling Pipeline

With reference to FIG. 1A, an automated, patient-specific head modeling pipeline is illustrated. FIG. 1A outlines a method for sEEG head modeling.

The computational pipeline of FIG. 1A is a platform where clinicians can input patient specific neuroimaging (e.g., T1 MRI, post-operative CT, pre-operative CT, diffusion weighted MRI, angiograms, contrast enhanced T1 MRI, T2 MRI, and other suitable imaging) and obtain a patient specific finite element model of the voltage distributions generated by every possible neural source in the brain. These voltages are necessary for any computational tool that builds on the electrophysiological recordings obtained through seizure monitoring.

In some embodiments of the modeling pipeline, DEETO is a software package utilized to automatically determine the electrode contact locations from a post-operative CT image to localize the electrode contacts. Novel software was developed to create the electrode geometries in MATLAB. The disclosed patient-specific head modeling pipeline is foundational software that will allow development for other computational tools and methods disclosed herein. In other words, the automated patient-specific head modeling platform of FIG. 1A can be used to generate the head models upon which additional tools and methods can be implemented.

In one embodiment, the method builds a patient specific anatomically realistic finite element model (FEM). T1-weighted MRI, Post-op CT, and DW-MRI images are segmented into five tissue-types: skin, skull, CFS, cortical grey matter, and white matter. Anisotropic conductivity tensors are defined to the mesh elements, using the following conductivity values for each respective tissue-type: 0.20, 0.01, 1.79, 0.23, and 0.15 S/m.

Embodiments of the present disclosure include a method for generating a stereo-EEG model including the steps of: receiving images of a patient brain; determining a coordinate for each of a plurality of electrodes; generating three-dimensional geometry of the patient brain based on the images; generating three-dimensional geometry of the plurality of electrodes within the three-dimension geometry of the patient brain based on the images and the coordinate for each of the plurality of electrodes; defining an electrical property of the three-dimensional geometry of the patient brain; and outputting a finite element model for the patient brain and the plurality of electrodes. In some embodiments, the contact locations are automatically determined from the post-operation CT.

As described further herein, in some embodiments, the method further comprises determining a transfer matrix that defines the voltages at the plurality of electrodes generated by a plurality of neural sources.

As described further herein, in some embodiments, the imaging comprises T1 MRI, post-operation CT, diffusion weighted MRI, angiograms, contrast enhanced T1 MRI, and/or T2 MRI.

As described further herein, in some embodiments, generating three-dimensional geometry of the patient brain comprises extracting a skin mesh, extracting a white matter layer, extracting a cerebrospinal fluid layer, extracting a dura layer, extracting a gray matter layer, and/or extracting a pial layer.

As described further herein, in some embodiments, defining the electrical property of the three-dimensional geometry of the patient brain comprises defining electrical conductivity for a plurality of tissue types.

As described further herein, in some embodiments, generating the three-dimensional geometry of the plurality of electrodes comprises using a transformation matrix to register electrode contact locations to the images.

As described further herein, in some embodiments, the coordinate is an entry coordinate and / or a target coordinate.

### 3. Brain Tissue Recording Sensitivity

Implanting the minimum necessary number of electrodes is important because each additional electrode increases the risk of hemorrhage, whereas too few electrodes can lead to improper localization and resection. The methods detailed herein use computational tools to simulate epileptic sources and find the optimal patient specific electrode configurations that records areas of interest with minimal numbers of implanted electrodes. By optimizing electrode placement and subsequently developing robust localization algorithms, we can make this procedure safer and more effective, increasing rates of seizure freedom and survival.

Currently, neurologists determine sEEG implantation trajectories by defining a region of interest (ROI) they believe contains the epileptogenic zone. They then maximize the number of electrode trajectories in their region of interest that avoid vasculature and critical structures. When neurologists see a spike in signal on an sEEG recording, they assume that the signal was generated by adjacent tissue less than approximately 5 mm away. In other words, existing methods rely on an assumption that recorded signals are generated from adjacent tissue less than approximately 5 mm away. However, by simulating epileptic activity using an anatomically realistic volume conductor head model, appreciable signals can be recorded up to 10 cm away for average sources (6 cm²). Thus, sEEG is not intrinsically localized. Based on this finding, an algorithm detailed herein quantify the recordable cortex of any given electrode configuration, which can be translated into a preoperational visualization tool for clinicians. The present disclosure provides efficient algorithms with parallel computing to generate a set of valid electrodes for any head model in less than approximately 24 hours. In some embodiments, a next-best tree search method is used to find top configurations to maximally map the region of interest (ROI) with the fewest number of electrodes. The resulting solution electrode configurations have higher recording sensitivities than the true clinician-generated implanted configurations for the same number of electrodes on a patient head model. Although large ROIs often cannot be fully mapped with a reasonable number of electrodes, they can map greater than approximately 90% before additional electrodes give only diminishing returns.

Existing algorithms find configurations that minimize risk and save time for clinicians, but do not optimize the recording sensitivity, or how well an electrode configuration can record from any epileptic source in the region of interest. The greater the recording sensitivity, the better that a configuration maps the ROI, or can record discernible voltages from all simulated epileptic sources in the region of interest. Optimal electrode configurations will have maximal recording sensitivity, mapping the whole ROI with at least two contacts on at least two electrodes, with the minimal number of electrodes. Existing software attempts to optimize recording sensitivity using a proxy measure of grey matter sampling ratio, which quantifies the percent of electrode contacts that are in gray matter, where seizures are generated. However, the existing method is agnostic to the actual area that each electrode can record from, and furthermore does not answer the question of how many electrodes are actually needed to map an area of interest. A simulation-based automated multiple trajectory planning presented herein determines safe and effective electrode trajectories to record from a ROI with the fewest electrodes.

To investigate the maximum distance away from a neural source that an electrode can record an appreciable signal, an anatomically realistic volume conduction head model is used to simulate the spatial distribution of voltages generated by epileptic sources. Then, efficient methods quantify the recording sensitivity of any electrode configuration and calculate the set of all valid electrode trajectories in the head. Finally, an optimization algorithm finds solution configurations for a variety of ROIs for one patient, finding better recording sensitives than clinician-generated implanted trajectories.

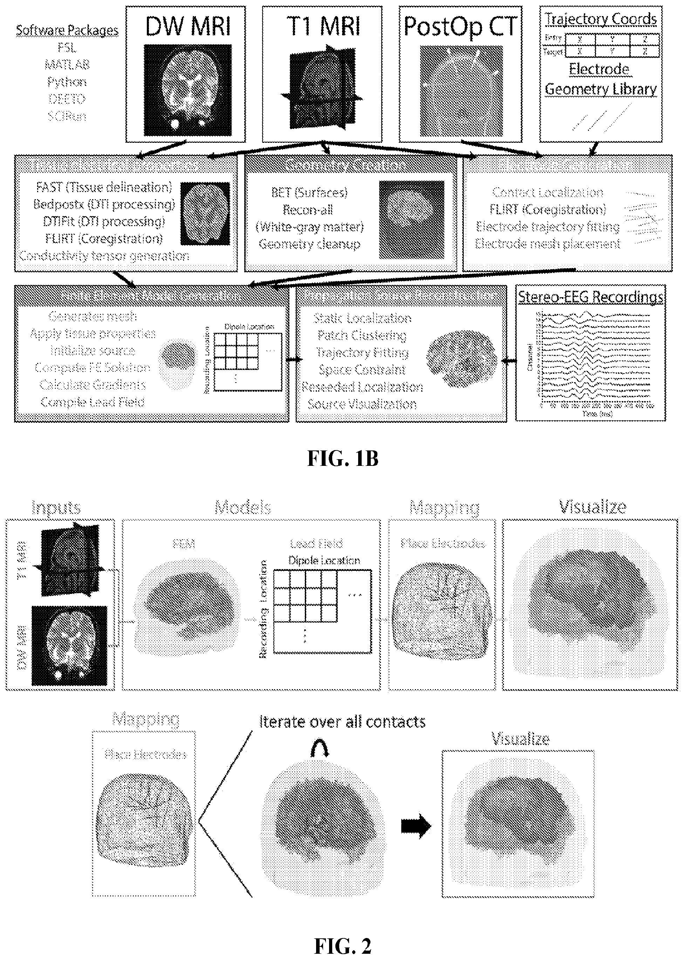

With reference to FIG. 2, a method for visualizing the brain tissue recordable by a set of sEEG electrodes is illustrated.

When determining the location of the epileptic source responsible for seizures, clinicians investigate the electrode recordings that have the highest amplitude epileptiform signals. Afterwards, clinicians determine the tissue that generates the signal to be within 5 mm of the contact that registered the high amplitude signal. However, this is a faulty assumption because the recording electrode is not necessarily within 5 mm of the signal generators and signal generators are spatially extended. The present disclosure provides a method to determine the tissue that is recordable by a set of sEEG electrodes. The method of FIG. 2 uses an estimate of the constant dipole moment density of the brain (e.g., source density) and scales it by the size of the active sources where large sources are made of many small sources. By calculating the voltages at every sEEG recording location generated by every possible dipole source in the brain, a map of the sources is created to recorded voltages for a patient. Patches of active cortex are then created at each possible location in the brain to account for the spatial extended nature of brain sources. By iterating through each electrode contact and determining the cortex that can be recorded, the tissue that is recordable is determined by a set of sEEG electrodes.

**Lead-field Generation and Discernible Distance Estimation:** To investigate the maximum distance away from a neural source that an electrode can record an appreciable signal, an anatomically realistic volume conduction head model is used to simulate the spatial distribution of voltages generated by epileptic sources. In some embodiments, a cortical surface mesh with approximately 40,000 elements is used to compute the spatial distribution of voltages generated by a single dipole current source at the center of each element in the cortical surface mesh, oriented orthogonal to the surface and pointing outward. The voltages at all points in a grid (e.g., a 2 mm grid) of the head are saved, and this resulting voltage field is defined as the "forward solution."

In some embodiments, the finite element method convergence is tested by comparing the voltages generated by two meshes of 19,593,867 and 63,925,906 elements. In some embodiments, an average of about 1 µV root mean squared error for single-dipole forward solutions generated using the 19,593,867-element mesh, which was consistent with the error using 37,356,840- and 52,031,029-element meshes. Thus, in some embodiments, the 19,593,867-element mesh is used in simulations to speed up computation.

After computing the forward solution for each single dipole, they are assembled together to form the lead-field matrix, which defines the transformation between the recorded voltages and all dipole sources. With about 466,779 recording locations and about 40,000 sources, the resulting lead-field matrix was approximately 160 GB using double-precision (64-bit) values. To reduce memory requirements but keep the accuracy of our recordable area measurements, in some embodiments, a compression scheme is utilized to use 16-bit integers. See Example 5.

Single dipole models have been shown to be accurate representations of epileptic sources using scalp recordings, but since sEEG records significantly closer to the sources (e.g., inside the brain) a more realistic extended dipole source model is used to simulate epileptic sources. In some embodiments, active sources are modeled as patches of triangles in a triangular surface mesh of the cortex, where each of 40,000 triangles contains a single dipole current source oriented orthogonal to the surface and pointing outward. In some embodiments, 1,000 spatially distributed sets of dipole current sources on the cortical surface-patch models-are used to simulate realistic epileptic sources. In some embodiments, patches are created by seeding 25 locations on each side of the cortex and iteratively recruiting adjacent mesh elements from the triangular cortical surface to build 20 patches of areas 0.1 to 30 cm² at each location, based on the area of cortex believed to be active during an interictal spike. For each patch, the generated voltages are calculated by finding the corresponding columns of the lead-field matrix and scaling the voltage values by the area of the corresponding triangle. Then, all the voltage values are summed and scaled by the established estimate of the dipole moment density (e.g., 1 nA-m/mm²). This results in the forward solution with the voltage at all points in a grid of the head generated by an active source, for each patch model. By calculating the distance from the source to the farthest point in the brain with discernible voltage (e.g., voltage above a baseline of noise), the distance of discernible signals (e.g., how far away signals can be recorded from a neural source) is determined. In some embodiments, the discernible voltage threshold is approximately 100 µV. As such, with this method, the recording sensitivity of any electrode configuration can be quantified.

**Quantification of Electrode Recording Sensitivity:** Given that appreciable signal can be recorded at significant distances away from epileptic sources, a visualization tool and metric is presented herein to quantify the recording sensitivity for any electrode or electrode configuration. The recording sensitivity is a measure of how well any contact location, electrode, or electrode configuration can record appreciable signal from realistic sources in the region of interest (ROI). To quantify the recording sensitivity for an electrode configuration, the recordable dipoles are calculated. In some embodiments, the recordable dipoles are the set of dipoles in the ROI that are mapped by at least two contacts on at least two electrodes. For a dipole to be "mapped" by a contact, the extended-dipole model of a specified patch area built around that dipole must generate appreciable signal (e.g., greater than approximately 100 µV) at that contact location. Thus, when all dipoles in the region of interest are mapped by at least two electrodes, the recording sensitivity is greatest.

To calculate the set of recordable dipoles for any electrode configuration, the set of voltages generated by a source at every dipole on the cortex is needed. Since extended dipole models are more accurate representations of epileptic sources than single dipoles for intracranial recordings, extended dipole models centered at every dipole in the cortical surface mesh are utilized to transform the lead-field matrix into a patch lead-field matrix. While the lead-field stores the voltages generated by every single-dipole model, the patch lead-field stores the voltages generated by an extended dipole source model of a given area built around every single element in the cortical surface mesh. Each column in the patch lead-field is a linear combination of the columns in the lead-field. Each row in the patch lead-field represents the voltages generated by all patch models at a single recording location in the brain.

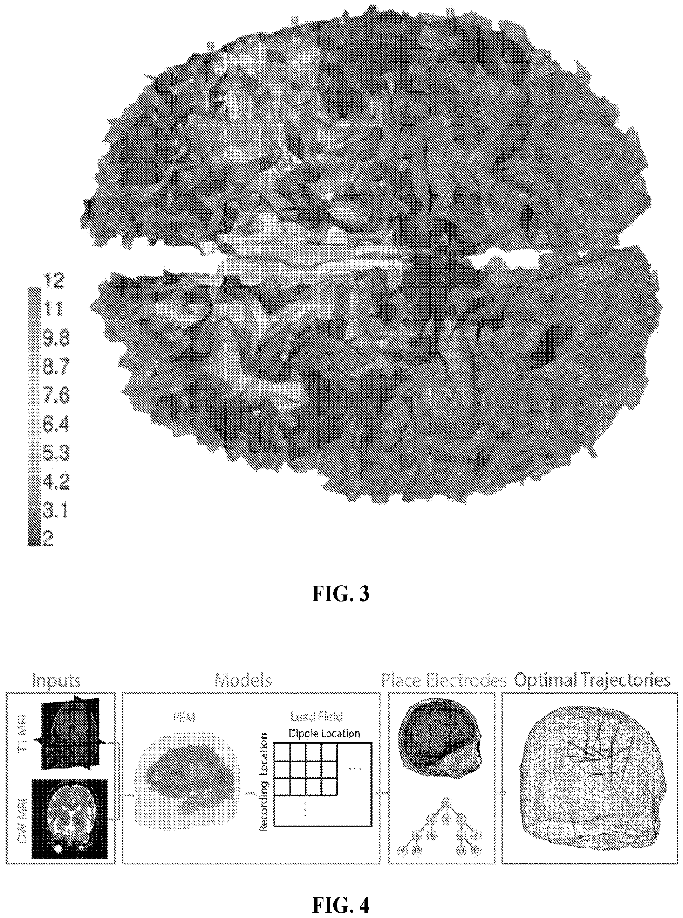

By thresholding each row in the patch lead-field by the measure of discernible voltage (e.g., greater than approximately 100 µV), the set of dipoles that are recordable by any contact location is determined. Then, given any electrode configuration, the extended dipoles that are recordable by at least two contact locations on at least two electrodes (e.g., the recordable area) are determined. The recordable area is visualized on the head model, where the color of each element corresponds to the number of electrodes that can record from it (FIG. 3). In some embodiments, the metric and computation methodology can be incorporated into a visualization tool for clinicians to plan and compare candidate electrode configurations.

The method disclosed herein uses finite element head modeling and extended dipole models to compute the patch lead-field matrix, and from this calculates the recordable dipoles and recordable area for any electrode configuration. This method can be used to visualize and quantify the recording sensitivity of any electrode configuration. As a preoperational tool, the method allows clinicians to visualize the recordable area of their implantation plans, comparing it to their region of interest, and they could add, remove, and move electrodes to make better configurations.

With reference to FIG. 3, the recordable area for a given set of electrodes can be visualized. The recordable tissue metric can be visualized, for example, on a 3D visualization of a patient's brain. FIG. 3 illustrates the number of contacts that map each cortical region. Uncertainty quantifications can also be shown with different dipole moment density estimates associated with the range of source strengths.

Embodiments of the present disclosure include a method for determining recordable brain tissue for a patient. The method comprises the steps of: generating a head model for the patient; determining a plurality of voltages throughout the head model that result from a plurality of neural sources; determining a recording sensitivity throughout the head model based on whether the voltages are above a threshold value for the plurality of electrodes; and displaying a visualization of the recording sensitivity throughout the head model.

As detailed herein, in some embodiments, determining the recording sensitivity throughout the head model is based on whether the voltages are about the threshold value for a user-defined number of contacts (e.g., 1, 2, 3, etc.) on each of the plurality of electrodes.

### 4. Optimized Electrode Implantation Trajectories

Clinicians can utilize the visualization method of FIG. 2 in combination with the disclosed automated head modeling platform (FIGS. 1A, 1B) to aid in planning sEEG implantation procedures.

When determining where to implant sEEG electrodes into a patient's head, a clinician plans trajectories to avoid major vasculature, sulci, and ventricles while maximizing the number of electrodes placed in the regions of interest. This procedure is automated as disclosed herein by using computation by searching through a theoretical tree, where each additionally implanted electrode corresponds to another layer of the search tree. Previous automated implantation algorithms found the trajectories to maximize the distance from major vasculature, sulci, and ventricles while also intersecting a region of interest for a set number of electrodes. However, the existing algorithms don't compute the tissue each electrode could record. The disclosed implantation algorithm computes the tissue each electrode can record (i.e., the recording sensitivity) and minimizes the number of implanted electrodes necessary to record from the targeted tissue. Clinicians will be able to use the implantation trajectory planning algorithm to design possible implantation trajectories that optimally map a region of interest.

With reference to FIG. 4, a method for optimizing electrode implantation trajectories based on recordable tissue is illustrated (e.g., an implantation trajectory optimization algorithm). Models are generated with the methods of FIG. 1A, 1B, but electrodes are not defined in the model. Instead, the method solves for the voltages at every possible recording location in the head for every possible neural source. With reference to FIG. 5, a method for optimizing electrode configuration is illustrated.

**Computing Candidate Electrode Trajectories:** To find an optimal electrode configuration, the set of all possible valid electrode trajectories is determined first. In some embodiments, the valid entry locations are defined by generating a template of the scalp surface on the MNI standard brain and selecting all points within 5 mm after linearly transforming the MNI template to best fit the patient head model. In one embodiment, the method samples 808 insertion locations from this region, and then samples 366 of insertion angles for each, keeping the implantation angle less than 10 degrees from normal to the scalp. This resulted in 295,728 electrode "lines"-paths of electrode implantations defined by a scalp insertion location and an insertion angle, without a defined implantation depth. Then, these lines are discretized into individual electrode trajectories by sampling insertion depth, keeping the total trajectory length, the distance from the scalp to the distal end of electrode, less than 10 centimeters. Finally, we eliminated electrodes that intersected critical structures (e.g., sulci, the midline, secondary skull locations, etc.). The occurrence of intersection of electrodes with the sulci surface is calculated to avoid areas where large blood vessels are likely to be. To calculate intersections, a bounding volume hierarchy method is utilized where the minimum distance from the electrode to the surface is found using an efficient binary tree search. All electrodes that passed within 2.5 mm of the sulci surface or 4 mm of the skull (apart from insertion location) or the midline are eliminated as candidates. In one embodiment, after eliminating risky electrode trajectories, a set of 83,596 valid electrode trajectories were left.

Next, the occurrence of collisions between each pair of electrodes is calculated, which requires many dimensions of problem size reduction and optimizations to complete. See Example 6. In one embodiment, the final implementation took approximately 45 minutes to run on 400 cores of a computing cluster.

**Tree Search Optimization:** After computing the set of all valid electrode trajectories and the occurrences of collisions between every pair, the metric of recording sensitivity is used to search for the best electrode configuration to map the full ROI with the fewest number of electrodes. In some embodiments, the generation of an electrode configuration is conceptualized as a tree traversal through an N-ary tree, where each node represents a valid trajectory and each node has N children, the number of valid electrodes. The optimization method keeps stepping down and choosing next trajectories until the cost function is zero. In some embodiments, the cost function is a representation of how much of the ROI is recordable. Before choosing another electrode, the electrodes that would collide with any previously added electrodes are eliminated. In some embodiments, an integrated cost function is used to take multiple thresholds of recordability into account in order to preference configurations that map the ROI with greater strengths. The cost function at a single threshold represents the number of dipoles that have not been mapped fully. However, a double weight to the dipoles that have not been mapped at all is added in order to preference picking electrodes to map the ROI weakly before mapping it strongly. In one embodiment, the cost function for a single threshold is described by EQN. 1. [EQN. 1] $\begin{matrix}{C}_{thr} = 3 N - 2 \left(# {mapped}_{thr}\geq 1\right) - \left(# {mapped}_{thr}\geq 2\right) \\ for thr in \left[100, 500, 1000\right] μV\end{matrix}$

An optimal configuration will be a shortest root-to-leaf path in the tree, where the cost function is minimized with the fewest electrodes. For any configuration of two or more electrodes, it is computationally intractable to find the true best configuration because the number of paths grows exponentially with the number of electrodes. Thus, in some embodiments, next-best iterative tree searches are used to find solution configurations. At each level, the electrode to maximally decrease the cost function at the highest threshold (1000 µV) is picked. If there were ties between electrodes, the cost function at the second threshold (500 µV) can be utilized. If there were ties, the cost function at the lowest threshold (100 µV) can be utilized. This selects configurations that can record from the ROI with high strength, but still allows lower strength mappings if higher threshold options are not found.

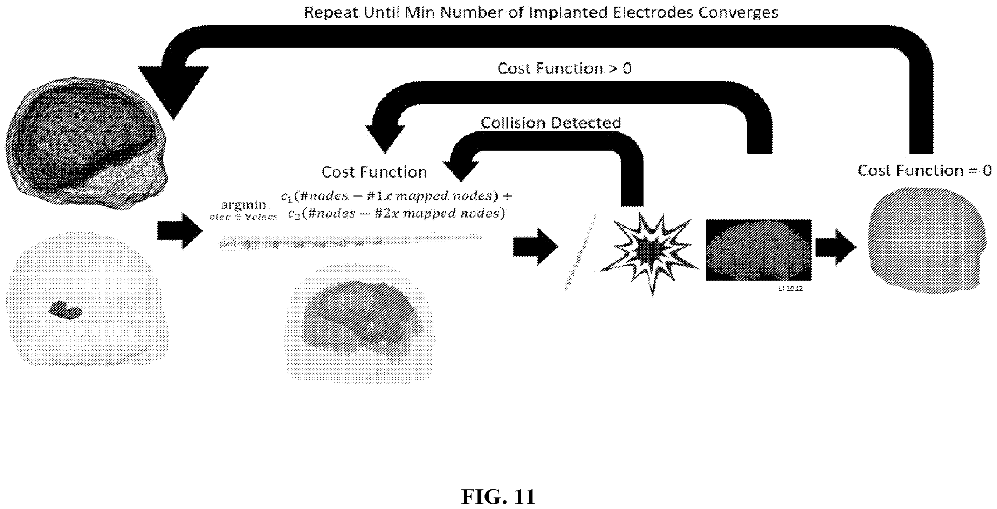

With reference to FIG. 11, a method for optimizing electrode implantation trajectories based on recordable tissue is illustrated. To map a given region of interest, the method iteratively seeds electrodes and determines if the region of interest is mapped using a cost function. In the illustrated embodiment, the cost function ensures that each node in the region of interest is mapped at least twice. The method of FIG. 11, omits trajectories that collide with vasculature and other electrodes. When the entire region of interest is mapped, a set of necessary electrodes is determined. This process is repeated until the minimum number of necessary electrodes stabilizes after, for example, 1000 searches.

Embodiments of the present disclosure include a method for determining implantation trajectories for a plurality of electrodes. The method comprises the steps of: generating a head model for the patient; determining a plurality of voltages throughout the head model that result from a plurality of neural sources; determining a recording sensitivity throughout the head model based on whether the voltages are above a threshold value for at least two contacts on each of the plurality of electrodes; and determining implantation trajectories for the plurality of electrodes to maximize recording sensitivity for a brain region of interest.

As described further herein, in some embodiments, determining implantation trajectories for the plurality of electrodes comprises eliminating possible trajectories based on a collision matrix.

As described further herein, in some embodiments, determining implantation trajectories for the plurality of electrodes comprises eliminating possible trajectories based on a position of a sulcus, a position of a blood vessels and/or a position of a ventricle.

As described further herein, in some embodiments, determining implantation trajectories comprises iteratively evaluating a cost function that encodes mapping every node in a portion of a cortical mesh at least once with a minimum resolution.

As described further herein, in some embodiments, iteratively evaluating the cost function stops when an improvement for adding an additional electrode is below a threshold.

### 5. Source Reconstruction

Source reconstruction algorithms are applied to stereo-electroencephalography (sEEG) signals where the electrodes are implanted into the brain and in close proximity to active neural sources. Existing approaches assume that the neural sources generating the sEEG signals can be localized using recordings from a single snapshot in time. However, interictal spikes and seizures are dynamic and propagate from one brain location to another over time. As used herein, "dynamic" source reconstruction is an encompassing term for any reconstruction that uses information from multiple times in the time series data. "Propagating" source reconstruction is only for source reconstruction where the source is moving. As used herein, propagating sources can be dynamic, but dynamic sources are not necessarily propagating. Additionally, there is information at one time point that can inform subsequent localizations. Thus, there is an ongoing opportunity for improvements to sEEG systems and methods.

Continuous sEEG recordings are obtained from the electrode contacts to allow clinicians to delineate the epileptogenic zone, the minimum amount of tissue needed to be resected to achieve seizure freedom. Epileptologists typically investigate the electrodes that exhibit patterns of activity indicative of the epileptogenic zone such as high frequency oscillations, seizure onset, and interictal spikes (synchronous brain activity between seizures). However, it is not feasible for the epileptologist to analyze completely the vast amount of data generated during seizure monitoring. Therefore, computational methods are necessary to distill the immense dataset into an interpretable set of signals. Current computational source localization algorithms for extracranial EEG yield source locations that alter resection planning for ~34% of patients. Little comparable source localization work has been done on sEEG signals because the sources are thought to be intrinsically localized to within 1 cm of the electrode contacts. However, as detailed herein, the neural signals can be recorded by electrodes up to approximately 10 cm away from the electrode contacts. Additionally, epileptic sources move in the brain over time, and some existing algorithms do not account for this phenomena. Most previous dynamic source reconstruction algorithms assumed that the recorded source is stationary and that the temporal dynamics of the sources can be used to refine the stationary source reconstruction estimate. The single propagating source reconstruction algorithm did not use estimates of the brain's anisotropy to conduct source reconstruction, but instead they developed a fitting algorithm based on the phase changes in seizures recorded by adjacent electrodes to reconstruct sources. This approach can localize a propagating source over time, but it cannot reconstruct the spatial extents. Therefore, a propagating source reconstruction algorithm is disclosed herein that use the common signals recorded by a set of sEEG electrodes and finite element head modeling to determine the size and location of the neural sources over time.

Stated another way, the goal of sEEG signal analysis is to delineate the epileptogenic zone. A clinician analyzes sEEG recordings from a patient by displaying all the recordings on a screen and investigating the electrode recordings that exhibit the highest amplitude epileptiform activity. They then delineate the epileptogenic zone by marking the tissue near the electrode contacts exhibiting the highest amplitude epileptiform activity. However, high amplitude recorded signals do not necessarily originate from the tissue immediately adjacent to the electrodes detecting the signals. Instead, these signals can originate from distant locations. Existing computational methods of source location and reconstruction are static meaning they localize using the neural signals generated at a single point in time.

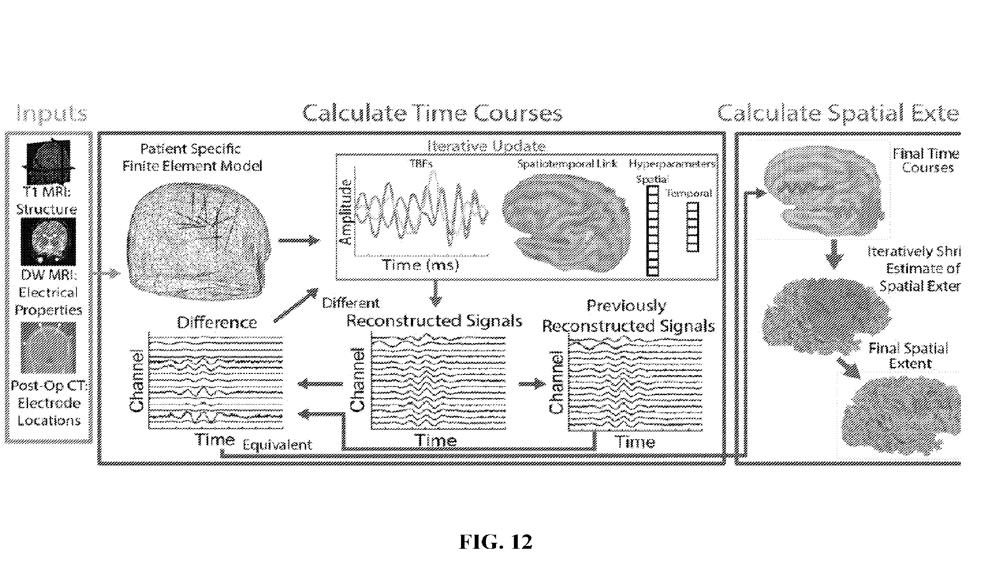

With reference to FIG. 12, an outline for a method of dynamic source reconstruction is illustrated. The head models are generated as outlined in FIGS. 1A, 1B. From the recorded sEEG signals, the method extracts a temporal basis function and parcellates the brain into functionally connected regions. Each iteration of the algorithm, the method updates the temporal basis function, spatiotemporal link, spatial hyperparameters, and temporal hyperparameters by minimizing free energy. When the reconstructed signals stop changing (e.g., the free energy converges), the method has generated a reconstructed time series. The time series is imported into another algorithm that iteratively shrinks the spatial extents of the source until the spatial extents converge. In the illustrated embodiment, the outputs of the method are the final time courses and spatial extents of the reconstructed sources (yellow boxes in FIG. 12).

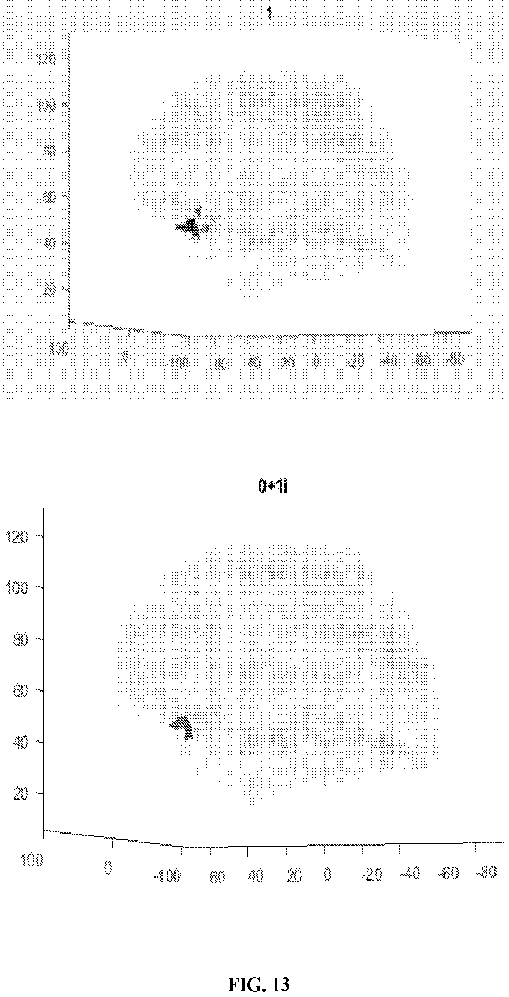

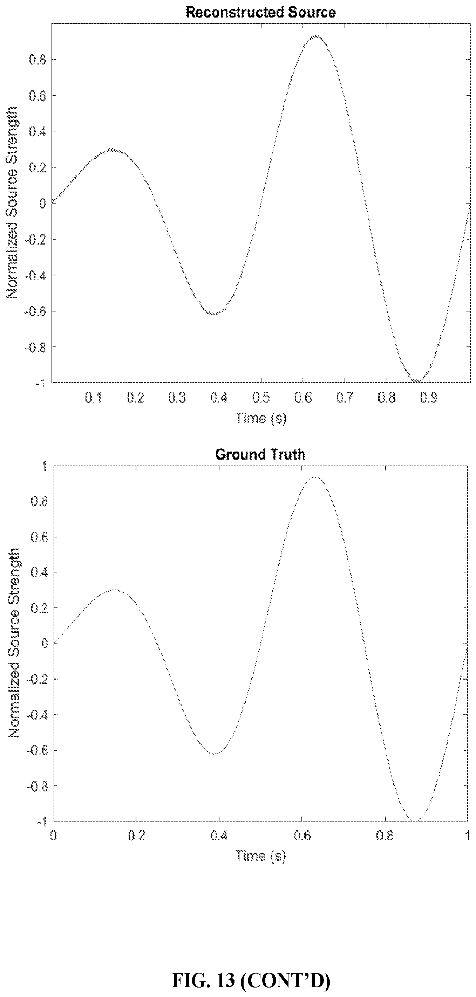

With reference to FIG. 13, the results from a synthetic source reconstructed with the method of FIG. 12 are illustrated.

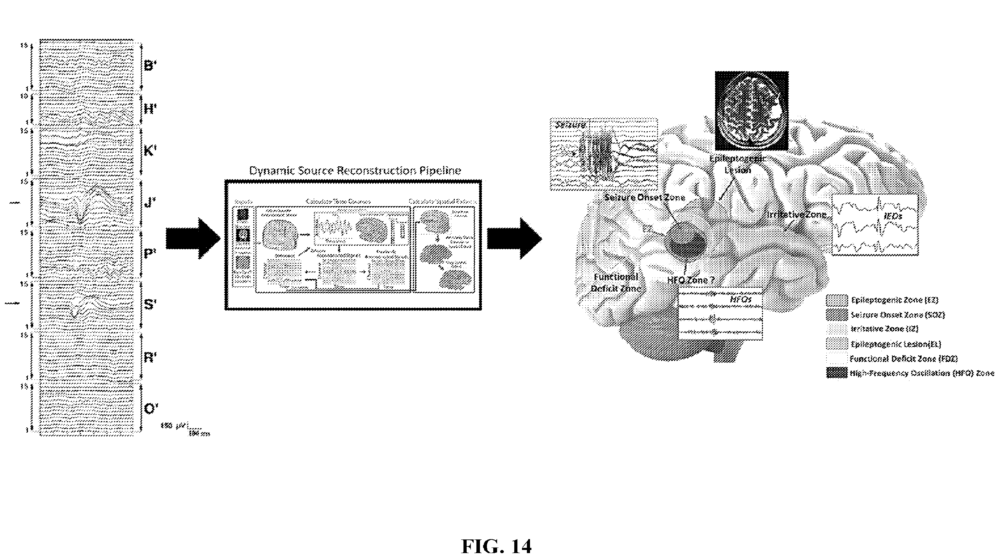

With reference to FIG. 14, a visualization output of the dynamic source reconstructed method of FIG. 11 is illustrated.

A sEEG localization algorithm according to one embodiment is dynamic, meaning it uses the information encoded in the temporal dynamics of a neural source to reconstruct the neural source. Neural sources are dynamic and the reconstruction at one point in time can inform the reconstruction at the next point in time. The dynamic reconstruction algorithm uses an input matrix that defines the recorded voltages generated by all possible neural sources to reflect the voltages recorded at sEEG locations based on the pipeline described hereinabove. In short, the dynamic source reconstruction method disclosed herein solves for stationary sources with a certain spatial extent using temporal dynamics of the sEEG recordings. In some embodiments, MATLAB code is translated into C and parallelized to allow efficient use of computational resources and improve computational performance. Input parameters are tuned to allow the algorithm to successfully reconstruct the spatial extents of synthetic neural sources with sEEG inputs. Dynamic source reconstruction improves localization of neural sources in EEG compared to static methods. However, no methods have previously been implemented on sEEG signals. Additionally, because sEEG electrode are much closer to the active neural populations and compared to EEG, a much higher (e.g., 100x) spatial resolution is used in the simulations compared to EEG-based solutions, in order to ensure proper source modeling. Therefore, the disclosed platform for source reconstruction has unprecedented spatial resolution for electrophysiological source reconstruction applications. In some embodiments, the source reconstruction is integrated into an analysis software package for sEEG signals to delineate different zones of epileptiform activity. This will allow clinicians to toggle the visibility (e.g., on a screen or display) of each of the reconstructed zones in a visualization of the signals: seizure onset zone, irritative zone, and high frequency oscillation zone. This gives clinicians another tool to analyze the signals and take advantage of additional information in sEEG signals that is currently unused.

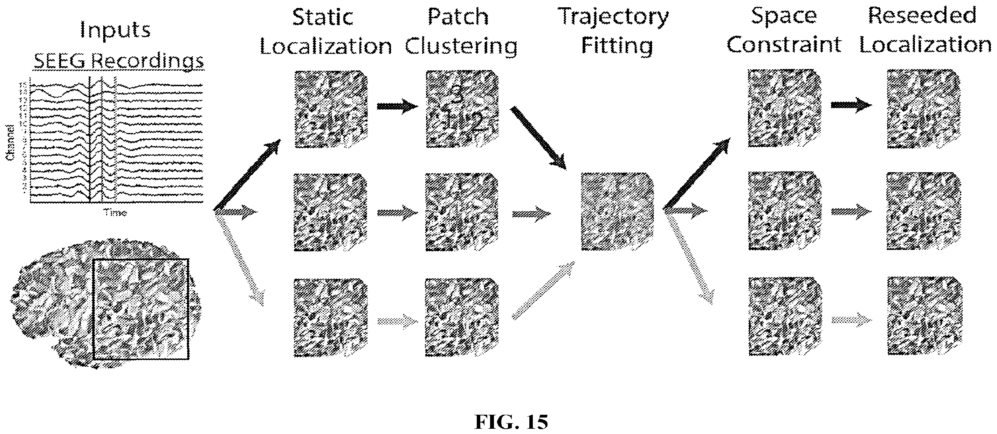

With reference to FIG. 15, an outline for a propagating source reconstruction method is illustrated that uses the lead field matrix, sEEG recordings, and a gradient matrix to reconstruct moving neural sources in time. The propagating source reconstruction method solves for propagating sources using the temporal dynamics to constrain the search space.

The lead field matrix is a transfer matrix that defines the voltages at all electrode contacts generated by all possible neural sources. The sEEG recordings are of epileptiform activity such as interictal spikes or seizures along with an epoch of resting activity used to define the noise floor. The gradient matrix defines neighboring elements in the brain mesh. In some embodiments, the propagating source reconstruction algorithm is built on top of a published static source reconstruction algorithm (IRES). IRES is a convex optimization algorithm, so improving the seeding of the initial conditions will lead to a better reconstructions.

With continued reference to FIG. 15, the propagating source reconstruction algorithm disclosed herein has 5 steps: (STEP 1) static localization, (STEP 2) patch clustering, (STEP 3) trajectory fitting, (STEP 4) space constraint, and (STEP 5) reseeded localization. In static localization, the search space is uniformly seeded and a static source localization algorithm (e.g., IRES) runs for each time point of our recorded neural activity. This outputs independent reconstructions for each time point of the recording. In patch clustering, each spatially discrete source is first clustered and labeled at each time point. Then each source over time is examined and relabeled sources that had more than 10% spatial overlap between time points to have the same label. In some embodiments, sources that were not present on more than 2.5 ms of consecutive reconstructions are filtered out, eliminating spurious noise sources. In trajectory fitting, filtered sources that generate continuous trajectories in time are isolated. The center of mass of each of each generated source is determined and a trajectory for each set of sources over time is smoothed. In some embodiments, if any trajectories passed within 4 cm (2x the space constraint size) of another trajectory, the trajectory that accounted for fewer time points is eliminated. The trajectories left are interpolated to fill in the spatially separated trajectories associated with each propagating source in the brain. In space constraint, all the brain elements that were within 2 cm of the fitted trajectory at each associated time point are isolated.

Finally, in reseeded localization, the source amplitude is scaled to minimize the least squares error between the original and reconstructed sEEG signals. Then, a static source localization algorithm (e.g., IRES) is run using the seeded initial source estimate and weighting matrices found from the scaled sources generated by space constraint. This leads to a new set of reconstructions that in a preliminary run of a single patient were improved compared to the original IRES simulation on all reconstruction metrics of Jaccard loss, % mapping of the simulated source, and localization error.

Embodiments of the present disclosure include a method for reconstructing propagating neural activity with time-dependent stereo-EEG data from a plurality of electrodes. The method comprising the steps of: simulating a static source reconstruction using the set of time-dependent stereo-EEG data; clustering adjacent populations of active neural sources together to generate time-dependent active clusters of neural sources; fitting a spatial trajectory to the time-dependent active clusters; constraining a search region to cortical points within a threshold distance of the spatial trajectory; simulating a propagating source reconstruction based on the search region and the spatial trajectory of the time-dependent active clusters; and displaying the propagating source in a three-dimensional head model.

As described further herein, in some embodiments, fitting the spatial trajectory to the time-dependent active clusters includes smoothing the spatial trajectory of the time-dependent active clusters to introduce temporal dependance of one source location on another.

Embodiments of the present disclosure include a method for reconstructing dynamic stationary neural activity with time-dependent stereo-EEG data from a plurality of electrodes (not in accordance with the claimed invention), the method comprising: parcellating of a cortical mesh into synchronously active spatially disparate sources; iteratively solving for a temporal basis function underlying the activity of spatially disparate sources, wherein a spatiotemporal link defines which temporal basis function maps to which source, and wherein hyperparameters define the number of temporal basis functions and which spatially disparate sources are active; wherein convergence occurs when the difference between subsequent reconstructed sources is below a threshold; and the spatial extents of each source are iteratively shrunk using a stationary source localization algorithm with the solved time courses for the spatially disparate sources.

### 6. Methods and Systems

Embodiments of the present disclosure include a method including the steps of generating a patient specific head model; determining implantation trajectories for a plurality of stereo-EEG electrodes that maximize a recording sensitivity for a region of interest; implanting the plurality of stereo-EEG electrodes in a patient head based on the implantation trajectories (not in accordance with the claimed invention); recording time-dependent stereo-EEG data from the plurality of electrodes; reconstructing a dynamic source of neural activity based on the time-dependent stereo-EEG data; and displaying the dynamic source of neural activity in the patient specific head model.

As described further herein, in some embodiments, determining implantation trajectories includes using a visualization tool that displays recordable brain tissue.

As described further herein, in some embodiments, wherein reconstructing a dynamic source of neural activity includes reconstructing a plurality of dynamic sources of neural activity based time-dependent stereo-EEG data. The plurality of dynamic sources of neural activity includes, but is not limited to, interictal spikes, seizures, and/or high-frequency oscillations. displaying the dynamic source of neural activity includes individually toggling the display of a plurality of dynamic sources of neural activity.

The systems and methods described herein can be implemented in hardware, software, firmware, or combinations of hardware, software and/or firmware. In some examples, the systems and methods described in this specification may be implemented using a non-transitory computer readable medium storing computer executable instructions that when executed by one or more processors of a computer cause the computer to perform operations. Computer readable media suitable for implementing the systems and methods described in this specification include non-transitory computer-readable media, such as disk memory devices, chip memory devices, programmable logic devices, random access memory (RAM), read only memory (ROM), optical read/write memory, cache memory, magnetic read/write memory, flash memory, and application-specific integrated circuits. In addition, a computer readable medium that implements a system or method described in this specification may be located on a single device or computing platform or may be distributed across multiple devices or computing platforms.

One skilled in the art will readily appreciate that the present disclosure is well adapted to carry out the objects and obtain the ends and advantages mentioned, as well as those inherent therein. The present disclosure describes embodiments, which are exemplary, and are not intended as limitations on the scope of the present disclosure. Changes therein and other uses will occur to those skilled in the art.

### 7. Examples

Having now described the present disclosure in detail, the same will be more clearly understood by reference to the following examples, which are merely intended only to illustrate some aspects and embodiments of the disclosure, and should not be viewed as limiting to the scope of the disclosure.

The present disclosure has multiple aspects, illustrated by the following non-limiting examples.

### Example 1

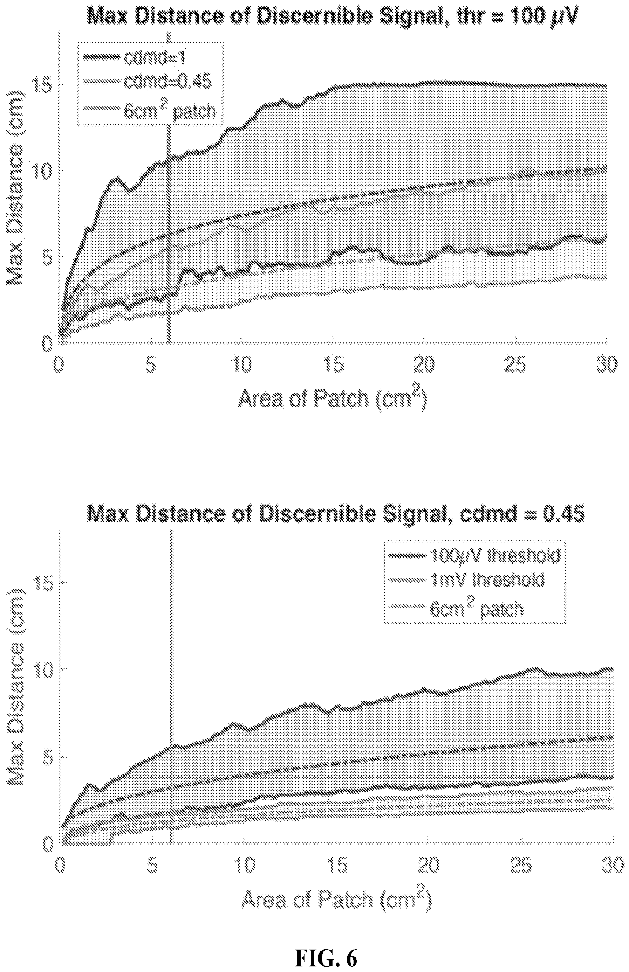

With reference to FIG. 6, results from optimizing sEEG electrode configurations are illustrated.

**Discernible distance:** the maximum distance away from 1000 extended source models (0.1-30 cm²) that an appreciable signal can be recorded is calculated. For a 6 cm² source, discernible signals can be recorded up to approximately 10 cm away (FIG. 6). This distance, roughly 20 times the current assumption, suggests that each electrode can record from a significant area of the cortex. FIG. 6 illustrates the maximum discernible distance as a function of area of patch source. For a 6 cm² patch, signal can be recorded greater than approximately 10 cm away for a 100 µV threshold and a dipole moment density of 1 nAm/mm². However, this distance varies strongly based on the dipole moment density (A). For a lower dipole moment density and a 1000 µV threshold, discernible signals can be recorded less than approximately 1 cm away (B).

The distance varies significantly based on dipole moment density, discernible voltage cutoff, and area of active cortex. Even for the strictest parameters, however, discernible voltage can be recorded up to approximately 1 cm away from the active source, which is greater than the current clinical assumption of less than 5 mm.

**Discussion:** Appreciable signals can be measured a significant distance away from active sources. This means that sEEG is not intrinsically localization, and computational methods can measure and then optimize the recording sensitivity of electrode configurations. Efficient methods are detailed herein to simulate the lead-field matrix for a patient head model, quantify the recording sensitivity of any electrode configuration, and calculate all valid electrode trajectories for a given patient. In some embodiments, a next-best tree search is performed with an integrated cost function to find near-solution configurations for realistic ROIs. To map a ROI smaller than one hemisphere using proper configurations, any additionally implanted electrodes past 9 give only diminishing returns and may therefore be unnecessary. The methods disclosed herein find optimal electrode configurations that transform sEEG into a procedure based in science rather than guesswork, improving epileptogenic zone localization and increasing rates of seizure freedom.

With continued reference to FIG. 6, with the finding that for a 6 cm² source, discernible signals can be recorded up to approximately 10 cm away; each electrode can likely record from a truly significant area of the cortex. For epileptogenic zone localization, clinicians could perhaps use far fewer electrodes, making this a much safer operation. Furthermore, epileptic sources may be significantly smaller than commonly thought and this might enable clinicians to resect much smaller areas of brain tissue to give seizure freedom. This estimate of discernible distance, however, is dependent on the size and strength of epileptic sources in our model. This estimate assumes that any voltage of at least 100 µV in magnitude would be detectible above the sEEG noise floor. In some embodiments, larger voltage thresholds are set, especially if clinicians want to visually inspect the voltage spikes due to epileptic activity. Also, since discernible signal can be recorded at a significant distance away from active sources, the recording area is likely larger than, and distinct from, the source area. Given that the size of epileptic sources is often estimated by simply recording differential voltage on the cortex, the distinction between these two areas can be clarified and further investigated.

Large ROIs often cannot be fully mapped with a reasonable number of electrodes, the method disclosed herein show they can map > 90% with 100 µV thresholds and single-electrode mapping before additional electrodes give only diminishing returns. The next-best search algorithm can find near-solution configurations for realistic ROIs. Further, for a single patient, the solution configuration of the methods disclosed herein had a 20% higher recording sensitivity at the lowest threshold for the same number of electrodes compared to the true implanted configuration. This suggests that the next-best search algorithm works well to find near-optimal configurations, and that these methods have the potential to improve not only the safety but also the efficacy of sEEG configurations.

The application of the methods disclosed herein to the clinical setting relies on the development of robust localization algorithms to use sEEG data from likely fewer electrodes. This algorithm could utilize the high spatiotemporal resolution of the recordings to create a visualization of brain activation progressing through time. Ultimately, improving localization of the epileptogenic zone is imperative for improving success rates of epilepsy resection surgery.

### Example 2

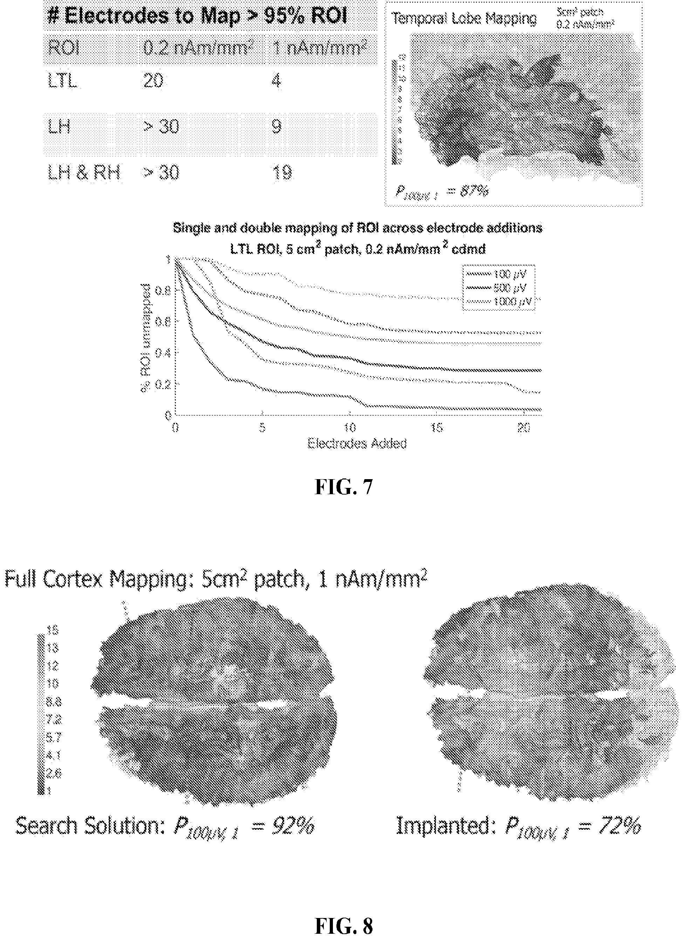

With reference to FIG. 7, the solution configuration generated by the method disclosed herein, shows greater recording sensitivity at 100 µV thresholds for single mappings than the clinician-generated and implanted configuration assuming a full cortex ROI.

**Solution Configurations:** the next-best search algorithm using an integrated cost function determines near-solution configurations for realistic ROIs. It is difficult to map the full ROI with double mappings, especially for our larger 500 µV and 1000 µV thresholds, using a reasonable number of electrodes. In some embodiments, the method determines how many electrodes would be needed to map 90-95% with single-electrode mapping and at the lowest threshold of 100 µV. While electrodes can keep being added to get slightly better recording sensitivities, the mapping power of each addition shown through convergence plots illustrate when additionally implanted electrodes would give only diminishing returns. The number of electrodes needed to map greater than 95% of the ROI for the left temporal lobe (LTL), left hemisphere (LH), and full cortex (LH & RH) is shown FIG. 7. The number of electrodes varies strongly based on the dipole moment density of our simulation methodology. In some embodiments, 1 nAm/mm² is used as the dipole moment density. A near-solution configuration for the LTL ROI is shown in FIG. 7 using a 0.2 nAm/mm² dipole moment density and a standard 5 cm² patch size. This configuration of 12 electrodes has a profit of 0.87, meaning it maps 87% of the ROI at the lowest threshold with single mappings. The generation of this configuration is visualized on a convergence plot (FIG. 7, bottom). This shows the iterative decrease in cost function for three different thresholds and both single and double mappings (solid and dotted lines, respectively). When about 12 electrodes are added, any further decreases in cost are minimal and would not be worth the increased risk of a larger configuration.

### Example 3

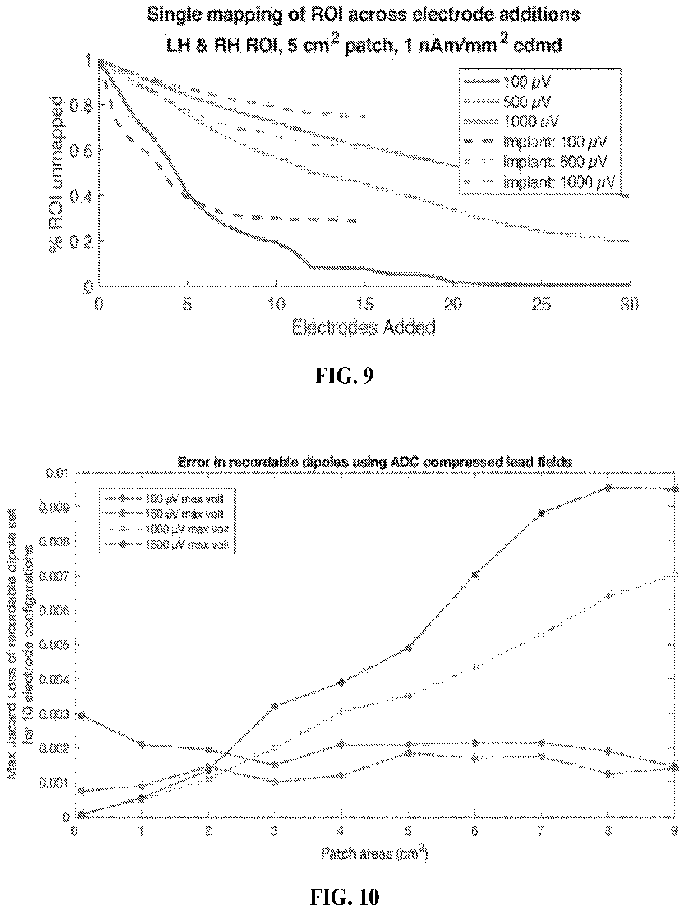

With reference to FIGS. 8 and 9, the solution configuration generated by the method disclosed herein, shows greater recording sensitivity at 100 µV thresholds for single mappings than the clinician-generated and implanted configuration assuming a full cortex ROI.

**Validation:** Given the near-solution configurations found with the next-best search algorithm, it was quantify how good these were compared to the configurations currently being used. The patient whose head model used in this example underwent sEEG implantation of 15 electrodes spaced throughout the cortex. Using the lead field simulations disclosed herein, the recording sensitivity is calculated for this truly implanted configuration and compared it to that of the full-cortex ROI solution configuration generated by the method. Using standard 5 cm² patches and a 1 nAm/mm² dipole moment density, the method solution configuration of also 15 electrodes mapped 92% of the ROI and the implanted configuration mapped 72%. Thus, for this example, the method solution configuration has higher recording sensitivity than the implanted configuration for the same number of electrodes (FIG. 8). The generation of these configurations are visualized with a convergence plot (FIG. 9). This shows the iterative decrease in three thresholds of the cost function (all for single mappings) for our solution and the implanted configuration, organized in the best possible way.

### Example 4

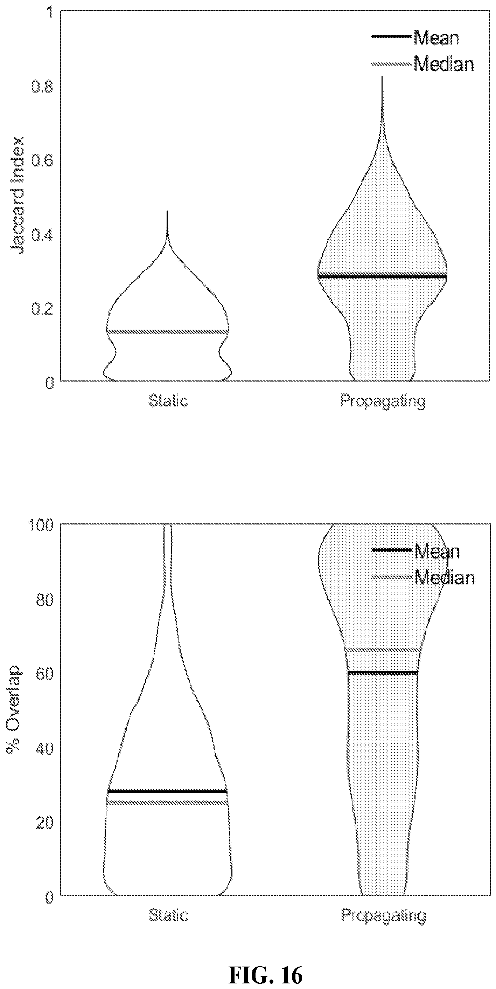

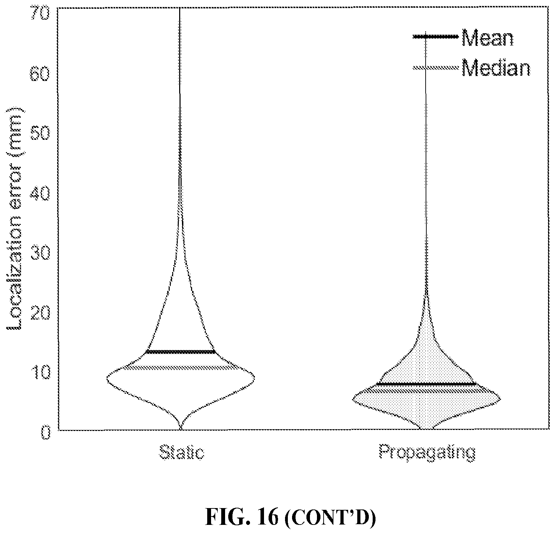

With reference to FIG. 16, reconstruction metrics of status and dynamic source reconstruction is illustrated.

**Rationale:** Existing source reconstruction algorithms approaches assumed that the neural sources generating the sEEG signals can be localized using recordings from a single snapshot in time. However, interictal spikes and seizures are dynamic and propagate from one brain location to another over time. Additionally, there is information at one time point that can inform subsequent localizations. Therefore, the propagating source reconstruction algorithm disclosed herein uses the recorded signal dynamics to reconstruct moving sources.

**Methods:** In some embodiments, the propagating source reconstruction algorithm is based on the static iterative reweighting edge sparse source reconstruction algorithm (IRES). IRES is a convex optimization algorithm that aims to minimize the strength and size of the source while ensuring that the reconstructed recordings do not differ greatly from the true recordings. IRES can be used to reconstruct independently sources at every time point of our sEEG recordings, which resulted in reconstructions that were in the neighborhood of the true sources. Then sources that were spatially connected are clustered and temporally overlapping to determine the trajectory of the source using each source's spatiotemporal center of mass. IRES reconstructions had many spurious and spatially discrete sources resulting in many trajectories. In the illustrated example, the trajectory that was active for the longest time is chosen and omitted trajectories that came within 4 cm of this trajectory. The trajectory was smoothed using a sliding window average with window size of 100 time points and generated search regions with a 4 cm radius centered at the trajectory. Since IRES is a convex optimization algorithm, initializing the algorithm well can greatly improve performance. Therefore, the trajectory defined regions are used to initialize a second run of the IRES algorithm to determine the final reconstructions of the moving sources.

Synthetic sources are created in three patient-specific head models with a left parieto-occipital implantation, a left temporal lobe implantation, and a bilateral exploratory implantation, where electrodes were placed throughout the brain. Moving synthetic sources are created in the region of interest for each patient and in the frontal lobe for the bilaterally implanted patient.

**Results:** The method disclosed herein reconstructs the moving synthetic source in each patient using the propagating source reconstruction method. The propagating source reconstruction method outperformed IRES on metrics of Jaccard index (0.28 vs 0.13), percent overlap with true source (28% vs 60%), and localization error (13.1 mm vs 7.6 mm) (FIG. 16).

**Conclusions:** Propagating source reconstruction method improves the reconstruction of neural sources relative to static reconstructions, and the disclosed method is generalizable across patients with varied implantation schemes.

### Example 5

**Compressed Lead-field Accuracy:** In some embodiments to calculate the recordable area for any electrode configuration, the full lead field matrix (e.g., the voltages generated at a source centered at every cortex location) is calculated. However, the full lead-field matrix, given 466,779 recording locations and about 40,000 sources, was about 160 GB large. To reduce memory requirements but keep the accuracy of our recordable area measurements, the necessary voltage resolution and range to be captured by a compression scheme was determined.

In some embodiments, to compress the lead-field, a linear analog-to-digital conversion is performed of the voltage space. In MATLAB, the original lead-field is stored as 64-bit double-precision floating point numbers that can capture fine resolution and a large voltage range from 10-308 to 10308 for both negative and positive numbers. This space is transformed by first clipping it to a restricted range defined by a maximum voltage value, dividing by this value, multiplying by the maximum integer value in our compressed space, and rounding all values to integers. Using this compression scheme with 16-bit integers, there are 215-1 values (32,767) that are linearly spaced across the clipped positive voltage range. Thus, the range of the clipped voltage space defines the resolution of our compressed space. Because of this tradeoff between resolution and range of our compressed lead-field, four values of maximum voltage are tested to find the best candidate-100, 150, 1000, and 1500 µV. Given the discernible voltage threshold of 100 µV, the larger voltage cutoffs can capture larger values of voltage but with lower resolution.

In order to test the accuracy of the compressed lead field in generating the recordable dipoles for any electrode configuration with any patch area, the recordable dipole sets are computed using both the compressed and the original lead-field for 10 patch areas (0.1-9 cm²) and 10 randomly generated electrode configurations from a set of valid electrodes. This was done using the same methods as the above, while now performing the digital-to-analog conversion by inverting the previous conversion process before thresholding. Accuracy was quantified by calculating the Jacquard Loss between the resulting dipole recordability sets, where the value at each dipole is 0 (it cannot be recorded by at least two contacts on two electrodes) or the number of electrodes over 1 that can record from the dipole with at least two contacts. The Jaccard Loss is a measure of dissimilarity between two sets and is defined as one minus the intersection over the union (EQN. 2). If the loss is 0, then it means the two dipole sets have exactly the same values, and if the loss is 1 it means the two dipole sets have none of the same values. [EQN. 2] $J \left(A, B\right) = 1 - \frac{\left|A∩B\right|}{\left|A∪B\right|}$

This captures not just the binary recordability of each dipole but also the degree of recordability-how many electrodes can record from it if it is recordable-to match the output resolution to what would be seen in the visualization tool and used in any optimization algorithm. Given the Jacquard Loss values for all configurations, patch areas, and voltage cutoffs, the maximum and average values were calculated for each patch area and voltage cutoff and visualized the resolution-range tradeoff (FIG. 10). After choosing the best max voltage cutoff and verifying the accuracy of our compressed lead-field, the metric of recording sensitivity is used to find the optimal electrode configuration for a given ROI.

With reference to FIG. 10, the maximum Jaccard loss of the recordable dipole sets is illustrated for 10 electrode configurations across 10 patch areas (0.1- 9cm²) using the compressed lead-fields with four different voltage cutoffs. The error in recordability reflects the resolution-range tradeoff, where smaller cutoffs sacrifice the ability to represent voltages larger than the discernible distance value (100 µV), seen with smaller patch areas, and larger cutoffs sacrifice the ability to represent small differences in voltage, seen with larger patch areas. The 150 µV cutoff balances the two considerations to minimize loss on both ends.

For a 150 µV maximum voltage, the compressed lead-field had a max Jacquard Loss of 0.002 across all areas and electrode configurations, establishing that it is very accurate in determining recordable dipoles compared to the full lead-field. With this value, voltage values of -150 µV to 150 µV are represented with 16-bit integers, giving a resolution of 4.6 nV. This allowed use of the compress full lead-field matrix from about 160 GB (as 64-bit double-precision floats) to about 40 GB, reducing memory usage by 75%.

### Example 6

**Computing Electrode Intersections:** For optimal electrode configurations, there cannot be electrode collisions. The occurrences of collisions between all pairs of valid electrodes are computed to prevent redundancy and allow efficient pruning of the search space. In some embodiments, electrodes are modeled as simple line segments. As such, electrode collisions are defined as any points along the electrodes passing within 4 mm. To compute all electrode collisions, N2/2 tests is performed because an N x N symmetric logical matrix represents the occurrences of collisions between N electrodes. With 83,596 candidate electrodes, this is a challenging task. However, given that conflicts are sparse and dependent on insertion location, the complexity of the problem is reduced by using a bounding volume hierarchy approach. Electrodes are grouped based on their insertion location and created bounding boxes around these clusters with a 2 mm buffer. If a pair of boxes do not overlap, no pair of electrodes within those two boxes can collide. Because most bounding boxes were geometrically separated, there were few overlaps and about 80% of the collision matrix could be zeroed. With the remaining electrodes, the occurrence of collisions are tested on the trajectory or "line" level to remove redundancy, as electrodes differing in depth but not in insertion location or angle overlap significantly. The minimum distance is calculated between points sampled from each trajectory length to determine which candidate pairs have conflicts.

**Electrode Collision Optimization:** Using high performance computing and algorithm optimizations, the collision matrix is calculated in less than one hour. In one embodiment, a method to calculate each of 83,5962/2 collisions using a bounding volume hierarchy method is utilized, but such a method would have taken more than one year to run completely. In another embodiment, the complexity of the task was reduced by splitting the electrodes into groups based on their insertion location and building bounding boxes against each group. The overlaps were computed between all pairs of bounding boxes. The majority of bounding boxes did not overlap, so about 80% of the collision matrix could be zero, bringing our estimated runtime down to 3 months. In another embodiment, a simpler single-collision computation method includes simply calculating the minimum distance between points along each electrode line segment. This method was faster than the bounding volume hierarchy method, which brought the estimated runtime down to 1 month. Given that electrodes with the same insertion location and angle, just with varied depth, overlap significantly, the redundancy and computed collisions in the electrode "line" basis were removed before transforming the results to the space of individual electrode trajectories. This brought the runtime down to about 1 week, and finally parallel computing with 400 CPUs allowed us to calculate all collisions for 83,596 electrodes in less than approximately one hour. Table 1 summarizes electrode collision computation method improvements. By removing redundancy, simplifying the single-electrode computation method, and using parallel computing - the collision computation process for 83,596 electrodes was reduced from approximately 1.5 years to less than approximately 1 hour.

**TABLE 1:**

| Collision Computation method improvement | Runtime |
|---|---|
| Bounding Volume Hierarchy method | 1.5 years |
| Bounding box complexity reduction | 3 months |
| Distance calculation collision method | 1 month |
| Remove redundancy | 1 week |
| Parallel computing | < 1 hour |

Various changes and modifications to the disclosed embodiments will be apparent to those skilled in the art.

## Claims

1. A method for reconstructing propagating neural activity with received time-dependent stereo-EEG data from a plurality of electrodes, the method comprising:
simulating a static source reconstruction using the set of time-dependent stereo-EEG data;
clustering adjacent populations of active neural sources together to generate time-dependent active clusters of neural sources;
fitting a spatial trajectory to the time-dependent active clusters;
constraining a search region to cortical points within a threshold distance of the spatial trajectory;
simulating a propagating source reconstruction based on the search region and the spatial trajectory of the time-dependent active clusters; and
displaying the propagating source in a three-dimensional head model.

2. The method of claim 1, wherein fitting the spatial trajectory to the time-dependent active clusters includes smoothing the spatial trajectory of the time-dependent active clusters to introduce temporal dependance of one source location on another.

3. The method of claim 1 or claim 2, wherein generating the three-dimensional head model comprises:
receiving images of a patient brain;
determining a coordinate for each of a plurality of electrodes;
generating three-dimensional geometry of the patient brain based on the images;
generating three-dimensional geometry of the plurality of electrodes within the three-dimensional geometry of the patient brain based on the images and the coordinates for each of the plurality of electrodes;
defining an electrical property of the three-dimensional geometry of the patient brain; and
outputting a finite element model for the patient brain and the plurality of electrodes.

4. The method of claim 3, wherein generating the three-dimensional head model further comprises determining a transfer matrix that defines the voltages at the plurality of electrodes generated by a plurality of neural sources.

5. The method of claim 3, wherein the imaging comprises T1 MRI, post-operation CT, diffusion-weighted MRI, angiograms, contrast enhanced T1 MRI and/or T2 MRI.

6. The method of claim 3, wherein generating the three-dimensional geometry of the patient brain comprises extracting a skin mesh, extracting a white matter layer, extracting a cerebrospinal fluid layer, extracting a dura layer, extracting a gray matter layer, and/or extracting a pial layer.

7. The method of claim 3, wherein defining the electrical properties of the three-dimensional geometry of the patient brain comprises defining electrical conductivity for a plurality of tissue types.

8. The method of claim 3, wherein generating the three-dimensional geometry of the plurality of electrodes comprises using a transformation matrix to register electrode contact locations to the images.

## Patentansprüche

1. Verfahren zum Rekonstruieren einer sich ausbreitenden neuronalen Aktivität mit empfangenen zeitabhängigen Stereo-EEG-Daten von einer Mehrzahl von Elektroden, wobei das Verfahren umfasst:
Simulieren einer statischen Quellenrekonstruktion unter Verwendung des Satzes von zeitabhängigen Stereo-EEG-Daten;
Clustern benachbarter Populationen aktiver neuronaler Quellen, um zeitabhängige aktive Cluster neuronaler Quellen zu erzeugen;
Anpassen einer räumlichen Trajektorie an die zeitabhängigen aktiven Cluster;
Beschränken einer Suchregion auf kortikale Punkte innerhalb eines Schwellenabstands von der räumlichen Trajektorie;
Simulieren einer Rekonstruktion einer sich ausbreitenden Quelle basierend auf der Suchregion und der räumlichen Trajektorie der zeitabhängigen aktiven Cluster; und
Anzeigen der sich ausbreitenden Quelle in einem dreidimensionalen Kopfmodell.

2. Verfahren nach Anspruch 1, wobei das Anpassen der räumlichen Trajektorie an die zeitabhängigen aktiven Cluster ein Glätten der räumlichen Trajektorie der zeitabhängigen aktiven Cluster umfasst, um eine zeitliche Abhängigkeit eines Quellenorts von einem anderen einzuführen.

3. Verfahren nach Anspruch 1 oder Anspruch 2, wobei das Erzeugen des dreidimensionalen Kopfmodells umfasst:
Empfangen von Bildern eines Patientengehirns;
Bestimmen einer Koordinate für jede von einer Mehrzahl von Elektroden;
Erzeugen einer dreidimensionalen Geometrie des Patientengehirns basierend auf den Bildern;
Erzeugen einer dreidimensionalen Geometrie der Mehrzahl von Elektroden innerhalb der dreidimensionalen Geometrie des Patientengehirns basierend auf den Bildern und den Koordinaten für jede der Mehrzahl von Elektroden;
Definieren einer elektrischen Eigenschaft der dreidimensionalen Geometrie des Patientengehirns; und
Ausgeben eines Finite-Elemente-Modells für das Patientengehirn und die Mehrzahl von Elektroden.

4. Verfahren nach Anspruch 3, wobei das Erzeugen des dreidimensionalen Kopfmodells ferner ein Bestimmen einer Transfermatrix umfasst, die die Spannungen an der Mehrzahl von Elektroden definiert, die durch eine Mehrzahl von neuronalen Quellen erzeugt werden.

5. Verfahren nach Anspruch 3, wobei die Bildgebung T1-MRT, postoperative CT, diffusionsgewichtete MRT, Angiogramme, kontrastverstärkte T1-MRT und/oder T2-MRT umfasst.

6. Verfahren nach Anspruch 3, wobei das Erzeugen der dreidimensionalen Geometrie des Patientengehirns ein Extrahieren eines Hautnetzes, ein Extrahieren einer Schicht weißer Substanz, ein Extrahieren einer Schicht aus Zerebrospinalflüssigkeit, ein Extrahieren einer Duraschicht, ein Extrahieren einer Schicht grauer Substanz und/oder ein Extrahieren einer Pialschicht umfasst.

7. Verfahren nach Anspruch 3, wobei das Definieren der elektrischen Eigenschaften der dreidimensionalen Geometrie des Patientengehirns ein Definieren einer elektrischen Leitfähigkeit für eine Mehrzahl von Gewebetypen umfasst.

8. Verfahren nach Anspruch 3, wobei das Erzeugen der dreidimensionalen Geometrie der Mehrzahl von Elektroden ein Verwenden einer Transformationsmatrix zum Registrieren von Elektrodenkontaktpositionen an den Bildern umfasst.

## Revendications

1. Procédé de reconstruction d'une activité neuronale en propagation à l'aide de données de stéréo-EEG dépendantes du temps reçues en provenance d'une pluralité d'électrodes, le procédé comprenant :
la simulation d'une reconstruction de source statique à l'aide de l'ensemble de données de stéréo-EEG dépendantes du temps ;
le regroupement de populations adjacentes de sources neuronales actives pour générer des amas actifs dépendants du temps de sources neuronales ;
l'ajustement d'une trajectoire spatiale aux amas actifs dépendants du temps ;
la restriction d'une région de recherche à des points corticaux situés à moins d'une distance seuil de la trajectoire spatiale ;
la simulation d'une reconstruction de source en propagation sur la base de la région de recherche et de la trajectoire spatiale des amas actifs dépendants du temps ; et
l'affichage de la source en propagation dans un modèle tridimensionnel de tête.

2. Procédé selon la revendication 1, dans lequel l'ajustement de la trajectoire spatiale aux amas actifs dépendants du temps comprend le lissage de la trajectoire spatiale des amas actifs dépendants du temps pour introduire une dépendance temporelle d'un emplacement de source par rapport à un autre.

3. Procédé selon la revendication 1 ou la revendication 2, dans lequel la génération du modèle tridimensionnel de tête comprend :
la réception d'images d'un cerveau de patient ;
la détermination d'une coordonnée pour chacune d'une pluralité d'électrodes ;
la génération d'une géométrie tridimensionnelle du cerveau du patient sur la base des images ;
la génération d'une géométrie tridimensionnelle de la pluralité d'électrodes à l'intérieur de la géométrie tridimensionnelle du cerveau du patient sur la base des images et des coordonnées pour chacune de la pluralité d'électrodes ;
la définition d'une propriété électrique de la géométrie tridimensionnelle du cerveau du patient ; et
la production en sortie d'un modèle par éléments finis pour le cerveau du patient et la pluralité d'électrodes.

4. Procédé selon la revendication 3, dans lequel la génération du modèle tridimensionnel de tête comprend en outre la détermination d'une matrice de transfert qui définit les tensions au niveau de la pluralité d'électrodes générées par une pluralité de sources neuronales.

5. Procédé selon la revendication 3, dans lequel l'imagerie comprend l'IRM T1, la TDM postopératoire, l'IRM pondérée en diffusion, des angiogrammes, l'IRM T1 à contraste amélioré et/ou l'IRM T2.

6. Procédé selon la revendication 3, dans lequel la génération de la géométrie tridimensionnelle du cerveau du patient comprend l'extraction d'un maillage de peau, l'extraction d'une couche de matière blanche, l'extraction d'une couche de liquide cérébrospinal, l'extraction d'une couche de dure-mère, l'extraction d'une couche de matière grise et/ou l'extraction d'une couche piale.

7. Procédé selon la revendication 3, dans lequel la définition des propriétés électriques de la géométrie tridimensionnelle du cerveau du patient comprend la définition d'une conductivité électrique pour une pluralité de types de tissu.

8. Procédé selon la revendication 3, dans lequel la génération de la géométrie tridimensionnelle de la pluralité d'électrodes comprend l'utilisation d'une matrice de transformation pour recaler des emplacements de contacts d'électrodes sur les images.
